# EUROPEAN PATENT SPECIFICATION

(11) **EP 0 543 970 B1**
(45) Date of publication and mention of the grant of the patent: **28.08.1996**
(21) Application number: 92912229.9
(22) Date of filing: 08.05.1992
(51) Int. Cl.: C07C 29/141, C07C 31/20, C07C 45/75, C07C 47/19, C07C 69/675, C07C 67/44

(54) **MANUFACTURE OF NEOPENTYL GLYCOL (IIA)**
HERSTELLUNG VON NEOPENTYLGLYCOL (IIA)
FABRICATION DE NEOPENTYLGLYCOL (IIA)

(30) Priority: 17.06.1991 US 716177
(43) Date of publication of application: 02.06.1993
(73) Proprietor: Aristech Chemical Corporation, Pittsburgh, PA 15230-0250 (US)
(72) Inventor: SALEK, Jeffrey, S., Oakdale, PA 15071 (US); PUGACH, Joseph, Monroeville, PA 15146 (US)
(74) Representative: Grünecker, Kinkeldey, Stockmair & Schwanhäusser Anwaltssozietät
(86) International application number: US9203762
(87) International publication number: WO9222521

(56) References cited:
- FR-A- 2 297 202
- FR-A- 2 377 989
- GB-A- 1 017 618
- US-A- 4 219 508
- US-A- 4 851 592
- US-A- 4 855 515
- US-A- 5 004 839

## Description

This invention relates to the manufacture of neopentyl glycol. In particular it relates to the manufacture of neopentyl glycol by reacting isobutyraldehyde with paraformaldehyde in the presence of a catalyst comprising one or more oxides selected from the group consisting of cadmium oxide and yttrium oxide and triethylamine or other lower alkyl tertiary amines, and hydrogenating the resulting mixture of hydroxypivaldehyde (HPA) and
3-hydroxy-2,2-dimethylpropylhydroxypivalate, sometimes known as hydroxyneopentylhydroxypivalate (HNHP).

Prior to this invention, it has been known to make neopentyl glycol (2,2 dimethyl-1,3-dihydroxypropane, also known herein as NPG) by reacting formaldehyde with isobutyraldehyde (IBAL) and hydrogenating the resulting hydroxypivaldehyde (HPA). See U.S Patent 4,855,515, for example, which recites the historical development of the reaction and emphasizes the use of a particular catalyst in the hydrogenation step. U.S. Patent 3,808,280 discloses the use of triethylamine as a catalyst for the (aqueous) formaldehyde/IBAL reaction.

Each of the above references employs formaldehyde in the form of aqueous formaldehyde.

Paraformaldehyde is used in UK Patent 1,017,618 to react with IBAL in the presence of a tertiary amine to produce a reaction product containing apparently predominantly HPA which may be hydrogenated to neopentyl glycol.

WO 92/19579, which is a prior art document according to Article 54(3) EPC, discloses a method of making neopentyl glycol comprising reacting paraformaldehyde with isobutyraldehyde in the presence of a catalyst comprising a tertiary amine and one or more oxides of elements of Groups IB, IVA, IVB, VA, VB, VIB and VIII of the periodic table to obtain monomeric and dimeric hydroxypivaldehyde, and hydrogenating the monomeric and dimeric hydroxypivaldehyde by known chemical or catalytical methods.

It is the object of the present invention to provide a process for the production of neopentyl gylcol from isobutyraldehyde and paraformaldehyde, wherein the obtained mixture of HNHP and HPA can be subjected to the hydrogenation step without separation or purification.

Accordingly, the present invention provides a method of making neopentyl glycol comprising reacting paraformaldehyde with isobutyraldehyde in the presence of a catalyst comprising a tertiary amine and an oxide, selected from the group consisting of cadmium oxide and yttrium oxide, to obtain a reaction product containing hydroxypivaldehyde and at least 20% 3-hydroxy-2,2-dimethylpropylhydroxypivalate and hydrogenating the reaction product by
(a) mixing the reaction product containing hydroxypivaldehyde and at least 20% HNHP with at least 20% of an alcohol of the formula RR'CHOH, wherein R and R' are independently selected from the group consisting of hydrogen and alkyl groups having one to five carbon atoms, and
(b) contacting the mixture with hydrogen in the presence of a hydrogenation catalyst.

A specific reaction may be described as follows: The reaction is performed in a reflux apparatus wherein one equivalent of IBAL, one equivalent of paraformaldehyde, 0.01 equivalents of cadmium oxide, and 0.04 to 0.05 equivalents of triethylamine have been placed. The reaction mixture is stirred at the reflux temperature of IBAL (about 63-64°C) for one to six hours. The clear yellow molten liquid (a mixture of HNHP and HPA) is decanted from the cadmium oxide co-catalyst.

The HNHP/HPA mixture is, for example, hydrogenated by passing a methanol solution over a copper chromite catalyst at 100°-200°C and 3,55 to 20,79 MPa (500-3000 psig), to obtain the NPG, which is finally purified by recrystallization or distillation.

More generally, with one equivalent of IBAL there may be placed in a reaction vessel from 0.5 to 2 equivalents of paraformaldehyde, 0.001 to 0.1 (preferably 0.005 to 0.05) equivalents of cadmium oxide or yttrium oxide and 0.01 to 0.1 (preferably 0.02 to 0.08) equivalents of a tertiary amine. The reaction mixture is stirred until the desired conversation of IBAL is obtained. The resulting HNHP/HPA mixture containing at least 20% HNHP is readily hydrogentated.

As is known in the art, if the amine chosen has a boiling point lower than the boiling point (reflux temperature) of IBAL, pressure may be used.

The present invention provides a process in which water is minimized and is therefore relatively easier to perform since it does not require the separation and/or disposal of water; the process is also considerably more efficient than prior art processes, since the HNHP/HPA product can be used directly, i.e. without an arduous separation or purification process, for the hydrogenation step to NPG. Mild hydrogenation conditions, that is, temperatures as low as 100°C and pressures as low as 3.55 MPa (500 psig), may be used. The process is also more efficient in that fewer by-products are made and indeed one need not be concerned with the complications of by-products. Under properly controlled conditions, paraformaldehyde is easier and safer to store than aqueous formaldehyde. Substantially reduced emissions may be expected.

The metal oxide co-catalyst can be removed from the HNHP reaction product before it is hydrogenated, by filtration or any convenient means for recycling. The reaction may also be performed over a bed of catalyst.

Various tertiary amines can be used. Specifically, as catalysts any tertiary amines of the general formula R¹R²R³N, wherein R¹, R² and R³ are alkyl groups of the general formula C₁-C₁₅ and R¹ and R² may form a substituted or unsubstituted cyclic group having from 5 to 15 carbon atoms, can be used.

In the following the present invention is described in more detail by the following examples.

### Examples 1 to 16

### Production of the mixed HNHP/HPA product.

Table I recites the results of similar experiments utilizing:

| Reagent | Equivalents |
|---|---|
| IBAL | 1.00 |
| Paraformaldehyde | 1.00 |
| Triethylamine | 0.050 |
| Metal oxide | 0.010 |

With the exception of #16, the reactions were terminated 1 hour after the IBAL quit refluxing and then analyzed by G.C. Everything else was done as similarly as possible so that the effect of the metal oxides could be compared.

It will be seen that the selectivity for HNHP was quite striking in the cases of cadmium oxide and yttrium oxide. It will be noted from Example 16 that a relatively long reaction time favors the production of HNHP.

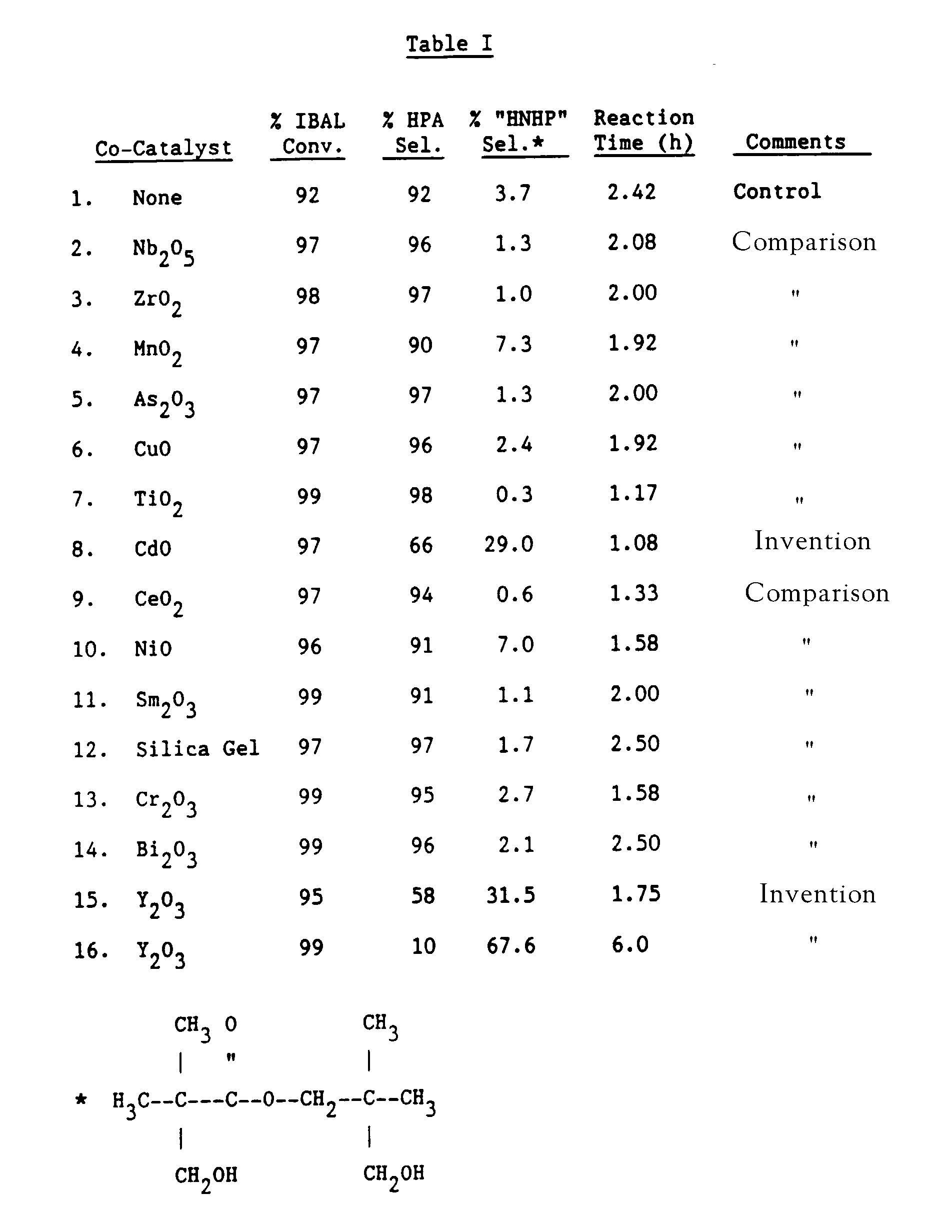

### Example 17

80.0 g of IBAL, 38.8 g of paraformaldehyde, 5.6 g of triethylamine, and 2.5 g of Y₂O₃ were charged with stirring into a 250 mL 3-neck roundbottom flask equipped with a reflux condenser and stirbar. The apparatus was lowered into a heated oil bath (80°C) giving moderate IBAL reflux within minutes. After 6h, the reaction mixture was filtered and diluted in 400 g of methanol. The reaction effluent was charged to an autoclave together with 16.0 g of CuCr₂O₄ and hydrogenated for 1.5h at 150°C followed by 1.5h at 180°C using 7.0 MPa (1000 psig) H₂. The results are summarized in Table II.

**Table II**

| *GC Analysis of Hydrogenated Effluent | | % HNHP Conversion |
|---|---|---|
| % isobutyl alcohol | 2.33 | 56.2% |
| % triethylamine | 5.42 | |
| % methyl hydroxypivalate | 16.12 | |
| % hydroxypivaldehyde | 0.00 | |
| % neopentyl glycol | 44.32 | |
| % NPG monoisobutyrate | 3.68 | |
| % hydroxyneopentyl hydroxypivalate | 25.77 | |
| % others | 2.36 | |

| | | |
|---|---|---|
| *GC area %'s are reported on a methanol-free basis. | | |

### Example 18

80.0 g of IBAL, 38.8 g of paraformaldehyde, 5.6 g of triethylamine, and 2.5 g of Y₂O₃ were charged with stirring into a 250 mL 3-neck roundbottom flask equipped with a reflux condenser and stirbar. The apparatus was lowered into a heated oil bath (80°C) giving moderate IBAL reflux within minutes. After 6h, the reaction mixture was filtered and diluted in 400 g of methanol. The reaction effluent was charged to an autoclave together with 16.0 g of CuCr₂O₄ and hydrogenated for 2h at 7.0 MPa (1000 psig) H₂ (sample A) followed by 2h at 13.9 MPa (2000 psig)H₂ using a temperature of 180°C (sample B). The results are summarized in Table III.

**Table III**

| *GC Analysis of Hydrogenated Effluent | | | % HNHP Conversion | |
|---|---|---|---|---|
| | sample A | sample B | sample A | sample B |
| % isobutyl alcohol | 1.74 | 2.56 | 51.6% | 84.3% |
| % triethylamine | 4.56 | 4.48 | | |
| % methyl hydroxypivalate | 14.27 | 23.89 | | |
| % hydroxypivaldehyde | 0.00 | 0.00 | | |
| % neopentyl glycol | 41.19 | 53.52 | | |
| % NPG monoisobutyrate | 2.63 | 1.38 | | |
| % hydroxyneopentyl hydroxypivalate | 33.31 | 10.77 | | |
| % others | 2.30 | 3.40 | | |

| | | | | |
|---|---|---|---|---|
| *GC area %'s are reported on a methanol-free basis. | | | | |

### Example 19

### HNHP hydrogenolysis compared to methylisobutyrate hydrogenolysis:

The following solutions were prepared:
(A)

| | |
|---|---|
| NPG | 47.6 wt.% |
| HNHP | 2.4 wt.% |
| triethylamine | 2.3 wt.% |
| methanol | 47.6 wt.% |

(B)

| | |
|---|---|
| methylisobutyrate | 5 wt.% |
| methanol | 95 wt.% |

A batch hydrogenation was performed on each solution using 1.4 wt.% CuCr₂O₄ at 150°C for 1h at 7.0 MPa (1000 psig) H₂. Ester hydrogenolysis was monitored. The results follow in Table IV. These results are surprising in that the ester impurities indigenous to the process in this invention are more easily hydrogenolyzed than a typical ester such as methylisobutyrate; they are also surprising in that we are able to hydrogenate easily at relatively low temperatures and pressures. This allows the recovery of high purity NPG product by simple distillation.

**Table IV**

| Ester | % Conversion |
|---|---|
| HNHP | 65.7 % |
| Methylisobutyrate | 1.4 % |

## Claims

1. Method of making neopentyl glycol comprising reacting paraformaldehyde with isobutyraldehyde in the presence of a catalyst comprising a tertiary amine and an oxide, selected from the group consisting of cadmium oxide and yttrium oxide, to obtain a reaction product containing hydroxypivaldehyde and at least 20% 3-hydroxy-2,2-dimethylpropylhydroxypivalate (HNHP) and hydrogenating the reaction product by
(a) mixing the reaction product containing hydroxypivaldehyde and at least 20% HNHP with at least 20% of an alcohol of the formula RR'CHOH,
wherein R and R' are independently selected from the group consisting of hydrogen and alkyl groups having one to five carbon atoms, and
(b) contacting the mixture with hydrogen in the presence of a hydrogenation catalyst.

2. The method of claim 1, wherein the tertiary amine is triethylamine.

3. The method of claim 1, including the step of recovering the oxide catalyst prior to hydrogentation of the 3-hydroxy-2,2-dimethylpropylhydroxypivalate/hydroxypivaldehyde mixture.

4. The method of claim 1, wherein the amine has the formula R¹R²R³N, wherein R¹, R² and R³ are alkyl groups of the general formula C₁-C₁₅ and R¹ and R² may form a substituted or unsubstituted cyclic group having 5 to about 15 carbon atoms.

5. The method of claim 1, wherein the alcohol is methanol.

6. The method of claim 1, wherein the hydrogenation is conducted at a pressure of 3,55 to 20,79 MPa (500 to 3000 psig) and a temperature of 100° to 200°C.

7. The method of claim 1, wherein the hydrogenation catalyst comprises copper chromite.

## Patentansprüche

1. Verfahren zur Herstellung von Neopentylglycol, umfassend die Umsetzung von Paraformaldehyd mit Isobutyraldehyd in Gegenwart eines Katalysators, umfassend ein tertiäres Amin und ein Oxid, ausgewählt aus der Gruppe, bestehend aus Cadmiumoxid und Yttriumoxid, um ein Reaktionsprodukt zu erhalten, das Hydroxypivaldehyd und mindestens 20% 3-Hydroxy-2,2-dimethylpropylhydroxypivalat (HNHP) enthält, und das Hydrieren des Reaktionsproduktes durch
(a) Mischen des Reaktionsproduktes, enthaltend Hydroxypivaldehyd und mindestens 20% HNHP, mit mindestens 20% eines Alkohols der Formel RR'CHOH, worin R und R' unabhängig voneinander ausgewählt sind aus der Gruppe, bestehend aus Wasserstoff und Alkylgruppen mit 1 bis 5 Kohlenstoffatomen, und
(b) Inkontaktbringen des Gemisches mit Wasserstoff in Gegenwart eines Hydrierungskatalysators.

2. Verfahren nach Anspruch 1, worin das tertiäre Amin Triethylamin ist.

3. Verfahren nach Anspruch 1, umfassend den Schritt der Rückgewinnung des Oxidkatalysators vor der Hydrierung des 3-Hydroxy-2,2-dimethylpropylhydroxypivalat/Hydroxypivaldehyd-Gemisches.

4. Verfahren nach Anspruch 1, worin das Amin die Formel R¹R²R³N besitzt, worin R¹, R² und R³ Alkylgruppen der allgemeinen Formel C₁-C₁₅ sind, und R¹ und R² können eine substituierte oder unsubstituierte cyclische Gruppe mit 5 bis etwa 15 Kohlenstoffatomen bilden.

5. Verfahren nach Anspruch 1, worin der Alkohol Methanol ist.

6. Verfahren nach Anspruch 1, worin die Hydrierung bei einem Druck von 3,55 bis 20,79 MPa (500 bis 3000 psig) und bei einer Temperatur von 100°C bis 200°C durchgeführt wird.

7. Verfahren nach Anspruch 1, worin der Hydrierungskatalysator Kupferchromit umfaßt.

## Revendications

1. Procédé de fabrication de néopentylglycol comprenant de faire réagir du paraformaldéhyde avec de l'isobutyraldéhyde en présence d'un catalyseur comprenant une amine tertiaire et un oxyde, choisi dans le groupe constitué de l'oxyde de cadmium et de l'oxyde d'yttrium, pour obtenir un produit de réaction contenant de l'hydroxypivaldéhyde et au moins 20 % d'hydroxypivalate de 3-hydroxy-2,2-diméthylpropyle (HNHP) et d'hydrogéner le produit de réaction en
(a) mélangeant le produit de réaction contenant l'hydroxypivaldéhyde et au moins 20 % de HNHP avec au moins 20 % d'un alcool de formule RR'CHOH, où R et R' sont choisis indépendamment dans le groupe constitué de l'hydrogène et des groupes alkyles qui ont de 1 à 5 atomes de carbone, et
(b) mettant en contact le mélange avec de l'hydrogène en présence d'un catalyseur d'hydrogénation.

2. Procédé selon la revendication 1, où l'amine tertiaire est la triéthylamine.

3. Procédé selon la revendication 1, qui comprend l'étape de la récupération du catalyseur oxyde avant l'hydrogénation du mélange d'hydroxypivalate de 3-hydroxy-2,2-diméthylpropyle/hydroxypivaldéhyde.

4. Procédé selon la revendication 1, où l'amine a la formule R¹R²R³N, où R¹, R² et R³ sont des groupes alkyles de formule générale C₁-C₁₅ et R¹ et R² peuvent former un groupe cyclique substitué ou non substitué ayant de 5 à environ 15 atomes de carbone.

5. Procédé selon la revendication 1, où l'alcool est le méthanol.

6. Procédé selon la revendication 1, où on réalise l'hydrogénation à une pression de 3,55 à 20,79 MPa (500 à 3 000 psig) et à une température de 100 à 200°C.

7. Procédé selon la revendication 1, où le catalyseur d'hydrogénation comprend du chromite de cuivre.
